# EUROPÄISCHE PATENTSCHRIFT

(11) **EP 0 749 290 B1**
(45) Veröffentlichungstag und Bekanntmachung des Hinweises auf die Patenterteilung: **19.02.2003**
(21) Anmeldenummer: 95942643.8
(22) Anmeldetag: 19.12.1995
(51) Int. Cl.: A61F 2/04, A61L 27/00

(54) **PLATZHALTER ZUM ANORDNEN IN EINER KÖRPERRÖHRE**
STENT TO BE SET INSIDE A BODY VESSEL
EXTENSEUR DESTINE A ETRE PLACE DANS UN VAISSEAU DU CORPS

(30) Priorität: 23.12.1994 DE 4446036
(43) Veröffentlichungstag der Anmeldung: 27.12.1996
(73) Patentinhaber: Alveolus, Inc., Charlotte, NC 28202 (US)
(72) Erfinder: Freitag, Lutz Dr., 58675 Hemer (DE)
(74) Vertreter: Bockermann, Rolf, Dipl.-Ing.
(86) Internationale Anmeldenummer: DE9501849
(87) Internationale Veröffentlichungsnummer: WO96019952

(56) Entgegenhaltungen:
- EP-A- 0 472 731
- EP-A- 0 621 015
- US-A- 5 330 500

## Beschreibung

Die Erfindung betrifft einen Platzhalter (Stent) zum Anordnen in einer Körperröhre gemäß den Merkmalen im Oberbegriff des Anspruchs 1.

Derartige Platzhalter werden bei der Behandlung von Stenosen eingesetzt. Eine Stenose ist eine angeborene oder erworbene Verengung in einer Körperröhre. Sie kann Hohlorgane, wie beispielsweise Luftröhre, Speiseröhre, Magen und Darm, oder Blutgefäße betreffen und als Folge von Krankheitsprozessen durch Geschwulstbildungen, Entzündungen mit bindegewebigen Wucherungen, oder schrumpfenden Narben, durch Thrombosen oder arteriosklerotische Prozesse sowie auch durch Verätzungen hervorgerufen werden. Bei entsprechender Ausprägung bewirkt eine Stenose in der betroffenen Körperröhre eine Stauung oder sogar den Verschluß derselben.

Es ist bekannt, zum Aufweiten bzw. Offenhalten von Stenosen, sei es in Blutgefäßen oder in anderen Hohlorganen, sogenannte Stents zu legen. Die Stents halten als innere Stütze das Gefäß offen und fungieren damit als Platzhalter.

Platzhalter (Stents) zum Offenhalten von Stenosen sind in vielfältigen Ausführungsformen bekannt. Es gibt sie aus Metall und/oder Kunststoffmaterial. Meist bestehen sie aus einem Geflecht von Metalldrähten, welche durch ihre Eigenspannung selbst expandieren. Es gibt aber auch solche Platzhalter, die erst an ihrem Einsatzort mittels einer geeigneten Vorrichtung, beispielsweise einem Ballonkatheter, in ihre aufgeweitete Stellung expandiert werden müssen.

Durch die DE-OS 42 19 949 oder die DE-OS 43 01 181 gehören auch Platzhalter aus sogenannten Memory-Metall zum Stand der Technik. Diese Platzhalter haben bei einer tiefen Temperatur einen geringen radialen Durchmesser. In diesem Zustand werden sie in der Stenose plaziert. Sie weiten sich dann beim Überschreiten einer Grenztemperatur, welche unter Körpertemperatur liegt, radial auf, so daß sie auf diese Weise eine Stenose offenhalten können.

Darüber hinaus ist es durch die EP 05 87 197 und auch die DE-OS 41 02 550 bekannt, die stützenden Metallsegmente in einen geschlossenen Mantel aus gewebekompatiblen Grundstoffen, beispielsweise Silikon, einzubetten, um so ein Durchwachsen der Platzhalter durch Gewebezellen zu verhindern.

Die vorgenannten Ausführungsformen verbindet jedoch der Nachteil, daß sich die Platzhalter, wenn radialer Druck auf sie ausgeübt wird, wie beispielsweise durch das Zusammendrücken vor dem Einsetzen, in axialer Richtung verlängern und dementsprechend beim Aufweiten wieder verkürzen.

Dieser Umstand muß insbesondere bei der Planung der Behandlung einer Stenose berücksichtigt- werden, da der Platzhalter sehr genau angepaßt sein muß. Eine nachträgliche Anpassung bzw. Korrektur der Länge im Körper ist nicht möglich.

Auch bei natürlichen Belastungen des Platzhalters, wie beispielsweise bei einem Hustenvorgang, ist eine Längenveränderung zu beobachten. Dies kann zu Problemen führen, weil es so zu Relativbewegungen zwischen dem Platzhalter und der Wand der Körperröhre in die der Platzhalter plaziert ist, kommen kann. Als Folge treten Reizungen der Röhrenwand oder des umliegenden Gewebes auf. Weiterhin kann die Relativbewegung auch zum Verrutschen des Platzhalters führen.

Aus der US-PS 50 64 435 ist zwar ein selbstexpandierender Platzhalter bekannt, der eine stabile axiale Länge gewährleisten soll. Dieser ist jedoch in seinem Aufbau vergleichsweise kompliziert. Er ist daher sowohl bei seiner Fertigung als auch in seiner Anwendung aufwendig. Darüber hinaus besteht bei dieser Form die Gefahr, daß das Maschengitter von Gewebezellen durchwachsen wird, was zu einem erneuten teilweisen oder vollständigen Verschluß des Gefäßes führen kann.

Ein selbstexpandierender Platzhalter, der mit einem Netz versehen ist, um zu verhindern, dass Gewebezellen in das Maschengitter einwachsen, ist aus der Patentschrift US-A-5 330 500 bekannt. Das Stützgerüst selbst besteht wie bei der vorliegenden Erfindung aus mehreren zick-zack-förmig geformten Drähten, wobei diese jedoch ringförmig angeordnet sind und durch Verbindungselemente zu einer geschlossenen, zylindrischen Struktur zusammengefügt werden.

Insgesamt gibt es eine Reihe von Platzhaltern, die die an sie gestellten Anforderungen je nach Anwendungsfall unterschiedlich erfüllen. Es besteht trotzdem das Bedürfnis die Platzhalter weiter zu entwickeln und zu verbessern. Insbesondere wird der Aufbau des Geflechts aus Metalldrähten, welche das Stützgerüst eines Platzhalters bilden, als zu kompliziert und damit nachteilig angesehen.

Der Erfindung liegt ausgehend vom Stand der Technik die Aufgabe zugrunde, einen Platzhalter mit einem einfachen Aufbau zu schaffen, bei dem insbesondere auch die Gefahr des Durchwachsens vermieden wird und der eine schonende Platzierung in der Körperröhre gewährleistet.

Die Lösung dieser Aufgabe besteht nach der Erfindung in den im kennzeichnenden Teil des Anspruchs 1 aufgeführten Merkmalen.

Kernpunkt der Erfindung bildet die Maßnahme das Stützgerüst des Platzhalters aus zick-zack-förmig geformten Drähten zu bilden. Jeder Draht weist mindestens drei Schenkel auf, wobei der mittlere Schenkel mit den beiden angrenzenden Schenkeln jeweils einen spitzen Winkel einschließt.

Die einzelnen Drähte des Stützgerüsts haben damit eine tannenbaumartige Konfiguration. Ein Schenkel geht unter Einschluß eines spitzen Winkels in einen gegenläufigen Schenkel über. An diesen Schenkel schließt sich dann ebenfalls unter einem spitzen Winkel der nächste Schenkel an.

Das aus mehreren versetzt zueinander angeordneten Drähten gebildete Stützgerüst ist in einen Mantel eingebettet, der aus einem bei Körpertemperatur elastischen Kunststoff besteht. Der Mantel verhindert, daß das Stützgerüst von Gewebezellen durchwachsen wird.

In seiner einfachsten Form kann ein ganzer Draht somit aus lediglich drei Schenkeln bestehen. Dadurch, daß der mittlere Schenkel mit den beiden angrenzenden Schenkeln jeweils einen spitzen Winkel einschließt, kann der mittlere Schenkel kürzer sein als die beiden angrenzenden äußeren Schenkel.

Eine vorteilhafte Ausführungsform des erfindungsgemäßen Grundgedankens wird in den Merkmalen des Anspruchs 2 gesehen. Danach sind die Schenkel eines Drahts so ausgelegt, daß jeweils zwei Schenkel einen Winkel einschließen, der kleiner als 45° ist.

Durch diese geometrische Form der Drähte ist eine Längenstabilität des Platzhalters gewährleistet. Selbst wenn der Platzhalter zusammengedrückt wird, ändert sich seine Länge nicht. Dies ist insbesondere bei der Planung der Behandlung einer Stenose von Bedeutung, da jetzt der Platzhalter exakt auf seine erforderliche Länge abgestimmt werden kann.

Wird auf einen solchen Platzhalter radial von außen Druck ausgeübt, so bewegt sich der zwischen zwei Schenkeln liegende Knickpunkt in einer drehförmigen Bewegung nach innen. Gleichzeitig bewegt sich jedoch das innenliegende Ende des Schenkels mit seinem Knickpunkt in einer gegenläufigen Bewegung nach außen. Dadurch heben sich die Bewegungen gegenseitig auf. Der Platzhalter bleibt in seiner Gesamtlänge stabil.

Auch wenn auf einen Draht, senkrecht zur Längsachse der Schenkel, Druck ausgeübt wird, bleibt der Platzhalter in seiner Länge stabil. In diesem Fall biegt sich der Draht zur Mitte hin durch. Die jeweils benachbarten Drähte würden sich dann relativ von einander entfernen. Dies wird jedoch durch die Einbettung des Stützgerüstes im elastischen Mantel verhindert.

Wenn eine Längenstabilität des Platzhalters nicht nötig oder nicht gewollt ist, kann der Platzhalter auch gemäß den Merkmalen des Anspruchs 3 so ausgebildet sein, daß der Winkel zwischen jeweils zwei Schenkeln größer oder gleich 45° und kleiner oder gleich 90° ist.

Ein solcher Platzhalter weist eine umfangseitige gleichmäßige Rückstellkraft auf. Er ist unkompliziert in seinem Aufbau und läßt sich dementsprechend einfach fertigen. Auch hinsichtlich der Konfektionierung, die auf die jeweilige Stenose abgestimmt sein muß, bringt der Platzhalter Vorteile mit sich, da man den Platzhalter einfach auf die erforderliche Länge zurechtschneiden kann.

Die umfangseitige Rückstellkraft des Platzhalters wird durch die Merkmale des Anspruchs 4 unterstützt. Danach greifen zwei umfangseitig des Mantels einander benachbarte Drähte verzahnungsartig ineinander. Das bedeutet, daß die jeweils von zwei Schenkeln eines Drahtes gebildeten Spitzen sich einander um ein bestimmtes Maß überlappen, und zwar in der Weise, daß die Spitzen eines Drahtes in die Ausbuchtungen des benachbarten Drahtes eingreifen. Die Drähte berühren sich jedoch nicht. Auf diese Weise wird ein effektives Zusammenwirken des Stützgerüstes mit dem Mantel gewährleistet. Wird auf den Platzhalter Druck ausgeübt, entfernen sich die Schenkel relativ zueinander, wodurch im Mantel eine Zugspannung entsteht, die die Rückstellung bewerkstelligt. Damit wird die Zugkraft im Mantel in eine Druckkraft transformiert. Diese steht der von außen einwirkenden Druckkraft entgegen und erzeugt somit eine Reaktionskraft in den Drähten.

Den Merkmalen des Anspruchs 5 entsprechend sind die jeweils benachbarten Schenkel über gerundete Längenabschnitte miteinander verbunden. Damit werden spitze Kanten vermieden, die u. U. zu Beschädigungen des Mantels führen könnten. Darüber hinaus läßt sich ein Draht dann durch Biegen über gerundete Formwerkzeuge auf einfache Weise herstellen.

Mit den Merkmalen des Anspruchs 6 wird ein Verrutschen des Platzhalters in der Körperröhre vermieden. Hierzu sind am Mantel über den Umfang verteilt Vorsprünge vorgesehen. Diese können gleichmäßig oder unregelmäßig angeordnet sein.

Obwohl es grundsätzlich denkbar ist, die Vorsprünge auf unterschiedlichste Weise zu erzeugen - beispielsweise durch Noppen, Haken oder Spitzen - wird eine günstige Ausbildung der Vorsprünge in den Merkmalen des Anspruchs 7 gesehen. Auf diese Weise werden über den Umfang des Platzhalters verteilt radiale Widerlager gebildet, die die ortsstabile Lage des Platzhalters gewährleisten.

Die Widerlager können dadurch erzeugt werden, daß jeweils zwei benachbarte Schenkel nicht planparallel zum Außenumfang des Platzhalters verlaufen, sondern hierzu relativ nach außen gekippt oder tordiert sind. Folglich erhält der Platzhalter eine nicht glatte, mit Vorsprüngen versehene Oberfläche. Diese Konfiguration ist insgesamt schonender für die betroffenen Organ- bzw. Gefäßwände und auch die Schleimhäute, als eine zirkuläre Verankerung. Die Oberfläche weist dann eine unregelmäßige geriffelte Struktur auf. Damit wird ein Abschnüren oder Abdrücken von Blutgefäßen auf dem Umfang des Platzhalters vermieden. Folglich ist eine bessere Blutversorgung in den außenliegenden Blutgefäßen gewährleistet.

Die Widerlagerstruktur des Drahtes kann auf seiner gesamten Länge vorgesehenen sein. Denkbar ist es aber auch, die Vorsprünge nur jeweils an einigen Schenkeln auszubilden.

Eine andere Möglichkeit einen Platzhalter mit Vorsprüngen zu schaffen ist gegeben, wenn man die Drähte des Stützgerüstes endseitig über den Mantel vorstehen läßt und/oder noch umformt. So können Vorsprünge in Form von endseitigen Krallen gebildet werden.

Nach dem Merkmal des Anspruchs 8 sind die Drähte aus Metall. Hier kann jedes Metall zum Einsatz kommen, das den Anforderungen aus medizinischer Sicht genügt.

Es ist denkbar, daß die Drähte aus Stahl bestehen und der Mantel aus einem Elastomer, wie dies Anspruch 9 vorsieht. Es muß sich um rostfreien Stahl handeln. Dieser ist preisgünstig und auch einfach in seiner Verarbeitung. Durch die dem Stahl innewohnende Eigenspannung wird der selbstexpandierende Effekt des Platzhalters gefördert. Als Material für den Mantel kommt bevorzugt Silikon zum Einsatz. Es können aber auch andere Synthese-Kautschukarten Verwendung finden, die die medizinischen Anforderungen, insbesondere hinsichtlich der Gewebeverträglichkeit und der Allergenfreiheit aufweisen. Durch die Auswahl des Elastomers ist es möglich die umfangseitigen Rückstellkräfte des Platzhalters mehr oder weniger stark einzustellen.

Für das Einsetzen des Platzhalters ist ein entsprechendes Werkzeug nötig. Das Werkzeug hält den Platzhalter beim Einführvorgang zusammen. Zum Plazieren wird der Platzhalter von dem Werkzeug freigegeben und expandiert in seine aufgeweitete Stellung allein durch die dem Material innewohnende Eigenspannung.

Die Drähte können aber auch aus einem Memory-Metall sein, wie dies Anspruch 10 vorsieht. Hierbei handelt es sich um eine Form-Gedächtnis-Legierung. Solche Legierungen werden meist aus einer Kombination der beiden Metalle Nickel und Titan gebildet (Nitinol).

Dieses Material hat bei einer tiefen Temperatur eine komprimierte Struktur. Es dehnt sich jedoch bei Überschreiten einer Grenztemperatur aus. Die jeweils gewünschten Grenzbedingungen können durch eine entsprechende Wahl der Legierungskomponenten eingestellt werden.

Als Material für den Mantel kommt ein Elastomer zum Einsatz mit einer geringen Zugkraft, wie dies beispielsweise Latex ist. Der Mantel muß die Expansion der Memory-Metall-Drähte zulassen und nach der Expansion eine solche Elastizität gewährleisten, daß ein Kollabieren, d. h. ein in sich zusammenfallen der Gesamtstruktur, verhindert wird. Das Elastomer bewerkstelligt folglich die ausreichende Stabilisierung der Drähte.

Auch für diese Kombination ist ein Einführwerkzeug notwendig.

Eine vorteilhafte Ausführungsform der Erfindung besteht in dem Merkmal des Anspruchs 11. Danach besteht der Mantel aus einem Memory-Elastomer. Dies ist ein temperaturabhängiges Elastomer (Shape Memory Polyurethan), welches in kaltem Zustand hart und klein ist. Folglich kann der Platzhalter problemlos eingeführt werden. Erst bei Körpertemperatur bzw. geringfügig unterhalb kommt es dann zur Expansion des Platzhalters.

Insbesondere die Kombination eines Memory-Elastomers als Mantel mit Drähten aus einem Memory-Metall, wie dies Anspruch 12 vorsieht, wird als besonders vorteilhaft angesehen. Die Drähte aus Memory-Metall bzw. Nitinol sind bei Raumtemperatur weich. Ein Shape Memory Polyurethan ist dagegen hart. Durch die Kombination ergibt sich ein Röhrchen, welches bei Raumtemperatur dünn und hart ist. Bei Körpertemperatur wird das Shape Memory Polyurethan weich und flexibel, während sich das Stützgerüst aus Nitinol entfaltet. Es entsteht dann ein dicker, harter, aber elastischer Platzhalter.

Ein Einführwerkzeug ist für einen solchen Platzhalter nicht erforderlich, weil der Platzhalter im kalten Zustand hart und klein ist. Er kann folglich problemlos eingeführt werden. Hierzu trägt auch bei, daß Nitinol im kalten Zustand verformbar, d. h. plastisch weich, ist.

Die Herstellung des Platzhalters erfolgt in der Weise, daß das Memory-Metall in einem Zustand, in dem es noch weich und formbar ist, mit dem Memory-Elastomer zusammengebracht wird, wenn dieses schon weich ist. Das Stützgerüst wird demnach in einem engen, kleinen Zustand in das Elastomer eingegossen. Der Herstellvorgang wird in einem Temperaturbereich durchgeführt, bei dem die beiden Materialien ihren Körperzustand noch nicht gewechselt haben.

Grundsätzlich ist es auch vorstellbar, daß die das Stützgerüst bildenden Drähte gemäß Anspruch 13 aus Kunststoff bestehen. Hierzu stehen geeignete Kunststoffe zur Verfügung, die die gewünschte Stabilität und Rückstellkraft für den Platzhalter gewährleisten.

Die Drähte aus Kunststoff können ebenfalls in einen Mantel eingebettet sein, der aus einem Memory-Elastomer besteht (Anspruch 14).

Eine weitere vorteilhafte Ausführungsform wird in dem Merkmal des Anspruchs 15 gesehen. Hiernach weisen die Drähte Sollbiegestellen auf. Auch auf diese Weise kann ein Platzhalter bereitgestellt werden, der sich durch seine Längenstabilität auszeichnet. Wird beispielsweise eine äußere Kraft senkrecht auf die von zwei Schenkeln gebildete Spitze aufgebracht, führt dies zu einer Durchbiegung der Schenkel. Die jeweils anderen Enden eines Schenkels bleiben jedoch in ihrer Lage ortsstabil, d. h. sie werden in axialer Richtung des Platzhalters gesehen, nicht verschoben. Der Platzhalter bleibt damit in seiner Gesamtlänge konstant. Die Rückstellung der Schenkel erfolgt durch die federnden Eigenschaften des Drahtes, welche durch die elastischen Eigenschaften des Mantels unterstützt werden. Die erfindungsgemäßen Sollbiegestellen können beispielsweise dadurch erzeugt werden, daß in den Drähten an geeigneten Stellen Verdünnungen oder Einkerbungen vorgesehen sind. Es ist aber auch möglich, die Sollbiegestellen durch die Kombination zweier oder mehrerer unterschiedlicher Materialien in den Drähten zu erzeugen.

Die Erfindung ist nachfolgend anhand von in den Zeichnungen dargestellten Ausführungsbeispielen näher erläutert.
Es zeigen:
- Figur 1: in der Seitenansicht einen Draht mit drei Schenkeln und der Darstellung von Bewegungsabläufen;
- Figur 2: einen erfindungsgemäßen Draht mit abgerundeten Knickpunkten;
- Figur 3a-g: sieben unterschiedliche Ausführungsformen von Drähten;
- Figur 4: einen Platzhalter in einer dreidimensionalen Darstellung;
- Figur 5a: einen abgewickelten Ausschnitt aus einem Platzhalter;
- Figur 5b: den abgewickelten Ausschnitt einer weiteren Alternative eines Platzhalters;
- Figur 6: in der Seitenansicht einen vertikalen Längsschnitt durch den Mantel eines Platzhalters;
- Figur 7: einen Ausschnitt eines Platzhalters im horizontalen Querschnitt;
- Fiaur 8a: zwei Schenkel eines Drahtes mit Soil-Biegestellen;
- Figur 8b: die Darstellung der Figur 8a in einer gebogenen Position
- Figur 9: einen Ausschnitt aus einem Draht, der aus zwei unterschiedlichen Materialien hergestellt ist.

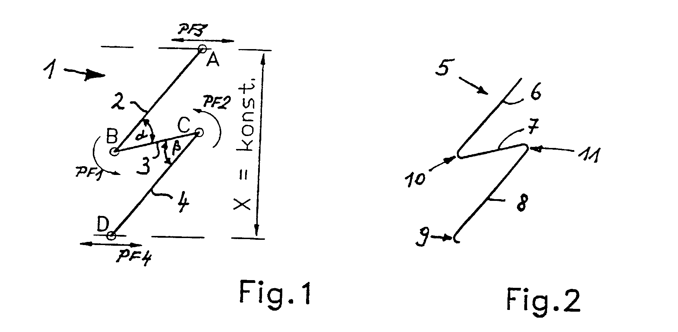

In der Figur 1 sind drei Schenkel 2, 3, 4 eines Drahtes 1 veranschaulicht. Der mittlere Schenkel 3 schließt mit den beiden angrenzenden Schenkeln 2 und 4 jeweils spitze Winkel α, β von 35° ein.

Die End- bzw. Knickpunkte zwischen den Schenkeln sind mit den Buchstaben A bis D gekennzeichnet.

Wird radial von außen auf den Knickpunkt B Druck ausgeübt und werden die Endpunkte A und D als fixiert angesehen, so bewegt sich der Knickpunkt B in einer drehfönnigen Bewegung (mit PF1 gekennzeichnet) in der Bildebene nach unten und innen. Gleichzeitig bewegt sich der innenliegende Knickpunkt C in einer gegenläufigen Bewegung (mit PF2 gekennzeichnet) nach außen bzw. oben. Durch die sich überlagernden Bewegungen heben sich diese gegenseitig auf. Die Gesamtlänge X vom Draht 1 bleibt folglich konstant. Die Endpunkte A und D können sich radial in der Richtung PF3 und PF4 bewegen, ohne eine axiale Verlagerung zu erfahren.

Bei der Belastung des Drahtes 1 mit einer Kraft, die senkrecht in die Bildebene gerichtet ist, würde sich der Draht 1 senkrecht zu seiner Längsachse durchbiegen.

Infolge der vorbeschriebenen Zusammenhänge kann eine Längenstabilität eines Platzhalters gewährleistet werden. Ein Platzhalter dessen Stützgerüst aus den zick-zack-förmig geformten Drähten (entsprechend der Konfiguration von Figur 1) aufgebaut ist, bleibt in seiner Gesamtlänge stabil, auch wenn er zusammengedrückt wird.

Die Figur 2 zeigt einen Ausschnitt aus einem Draht 5, dessen Schenkel 6, 7, 8 über gerundete Längenabschnitte 9, 10, 11 miteinander verbunden sind.

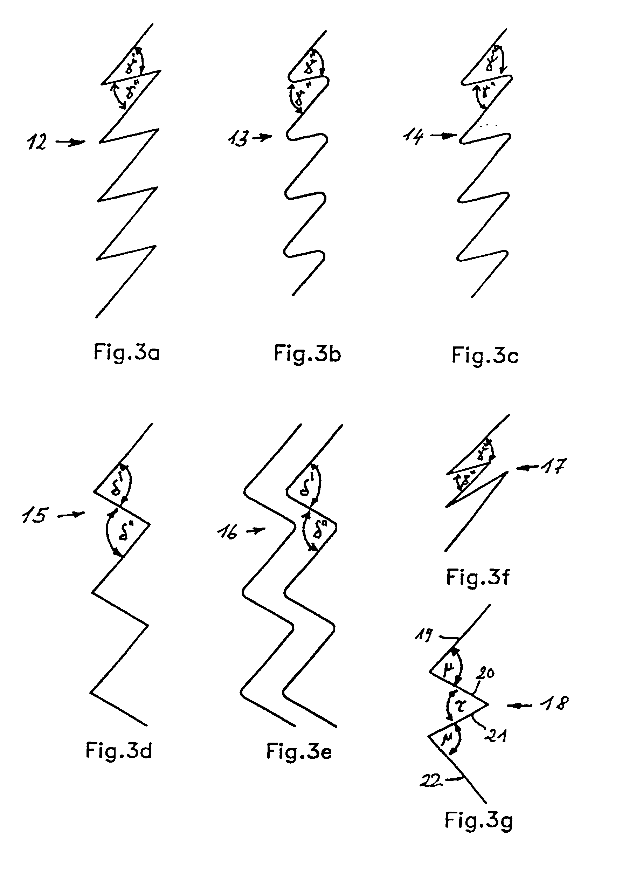

In den Figuren 3a bis 3g sind verschiedene Konfigurationen von Drähten 12 bis 18 dargestellt. Allen Drähten 12 bis 18 gemeinsam ist, ist daß die Schenkel jeweils spitze Winkel einschließen.

Hierbei sind die Winkel γ, γ'' der Drähte 12, 13, 14 und 17 jeweils kleiner als 45° . Die Winkel δ', δ" der Drähte 15 und 16 sind dagegen größer als 45°.

Die Figur 3g zeigt einen Draht 18 mit Schenkeln 19-22, bei dem die Winkel µ zwischen den Schenkeln 19 und 20 bzw. 21 und 22 jeweils größer als 45°, der Winkel τ zwischen den Schenkeln 20 und 21 hingegen kleiner als 45° ist.

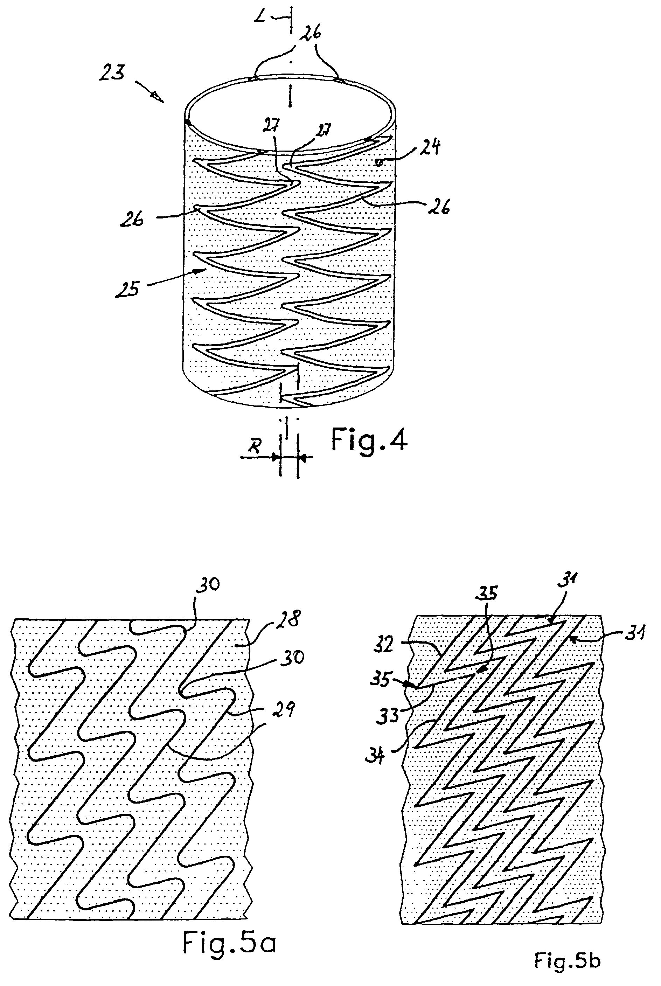

Aus der Figur 4 ist ein Platzhalter 23 mit einem hohizylindrischen Mantel 24 ersichtlich. In den Mantel 24 ist ein Stützgerüst 25 eingebettet. Das Stützgerüst 25 wird von den Drähten 26 gebildet, welche sich parallel zur Längsachse L des Mantels 24 erstrecken und jeweils zueinander versetzt sind.

Der Mantel 24 besteht aus einem Elastomer. Hierbei kommt bevorzugt ein Memory-Elastomer zur Anwendung. Die Drähte 26 sind aus Metall, wobei hier in vorteilhafter Weise ein Memory-Metall zum Einsatz kommt.

Die einzelnen Drähte 26 greifen mit ihren gerundeten Längenabschnitten 27 verzahnungsartig ineinander. Das bedeutet, die Längenabschnitte 27 überlappen sich in Bildebene gesehen um das Maß R.

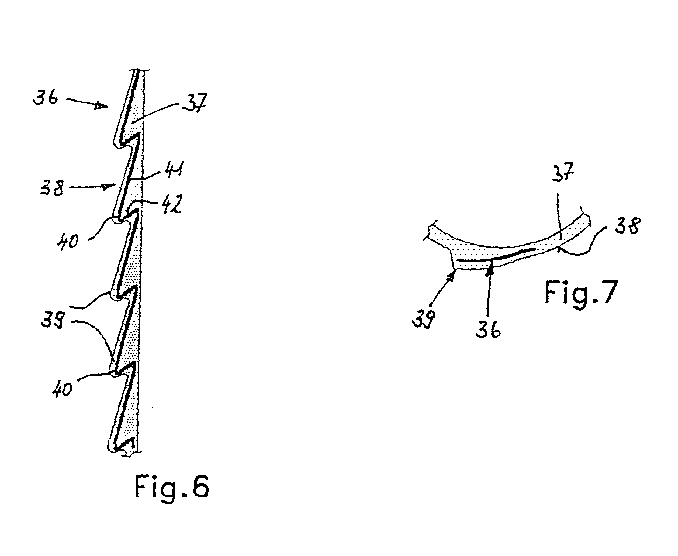

In der Figur 5a ist ein Ausschnitt aus einem Platzhalter dargestellt, bei dem im Mantel 28 Drähte 29 mit gerundeten Längenabschnitten 30 eingebettet sind. Auch die Drähte 29 greifen verzahnungsartig ineinander.

Eine Verzahnung ist auch bei der Konfiguration gemäß Figur 5b realisiert. Bei den hier eingesetzten Drähten 31 gehen jedoch die einzelnen Schenkel 32, 33, 34 mit spitzen Kanten 35 ineinander über.

Die Figuren 6 und 7 zeigen einen Draht 36, der im Mantel 37 über dessen Umfang 38 verteilt Vorsprünge 39 ausformt. Die Biegungen 40 zwischen den einzelnen Schenkeln 41, 42 des Drahtes 36 sind derart über den Umfang 38 nach außen tordiert, daß aus dem Mantel 37 die Vorsprünge 39 herausgebildet werden.

Die Vorsprünge 39 gewährleisten eine sichere Halterung eines Platzhalters nach seinem Einbau in einer Körperröhre.

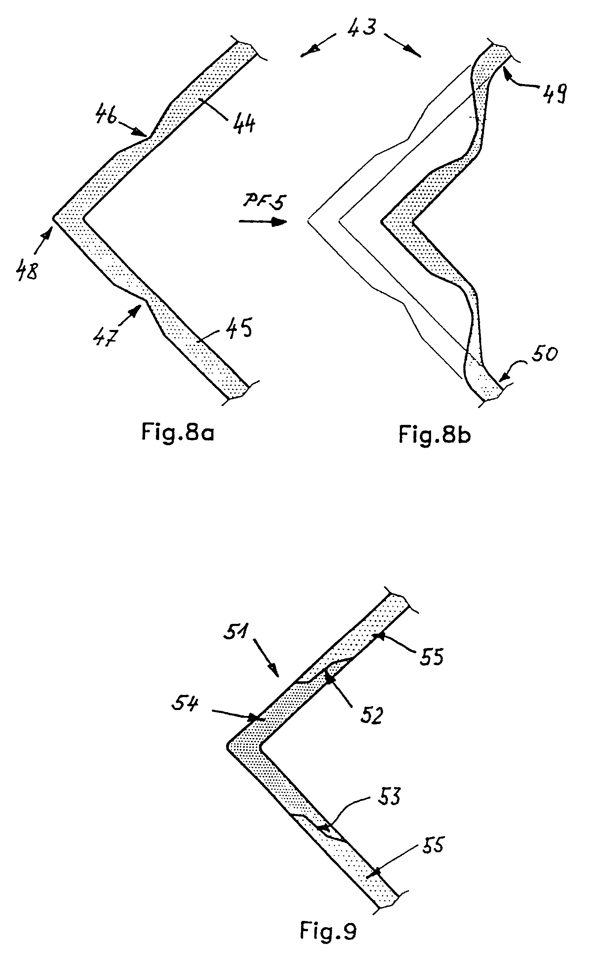

Die Figuren 8a und 8b zeigen einen Ausschnitt eines Drahtes 43, in dessen Schenkel 44, 45 Sollbiegestellen 46, 47 integriert sind. Wird der Draht 43 aus der mit PF5 gekennzeichneten Richtung belastet, wird die Spitze 48 zwischen den beiden Schenkeln 44, 45 in Bildebene nach rechts unter Verformung der Sollbiegestellen 46, 47 verschoben, ohne daß es zu einer Verlagerung der Schenkelbereiche 49, 50 kommt. Folglich bleibt der Draht 43 in seiner Gesamtlänge konstant.

Eine weitere Ausführungsform eines Drahtes 51 mit Sollbiegestellen 52, 53 zeigt Figur 9. Der Draht 51 weist Abschnitte 54, 55 auf, die aus unterschiedlichem Material bestehen. Durch die Kombination der verschiedenen Materialien mit unterschiedlichen Materialkenngrößen werden in dem Draht 51 Zonen realisiert, an denen der Draht 51 bei Belastung bevorzugt durchbiegt.

### Bezugszeichenaufstellung

- 1 -: Draht
- 2 -: Schenkel
- 3 -: Schenkel
- 4 -: Schenkel
- 5 -: Draht
- 6 -: Schenkel
- 7 -: Schenkel
- 8 -: Schenkel
- 9 -: Längenabschnitt
- 10 -: Längenabschnitt
- 11 -: Längenabschnitt
- 12 -: Draht
- 13 -: Draht
- 14 -: Draht
- 15 -: Draht
- 16 -: Draht
- 17 -: Draht
- 18 -: Draht
- 19 -: Schenkel
- 20 -: Schenkel
- 21 -: Schenkel
- 22 -: Schenkel
- 23 -: Platzhalter
- 24 -: Mantel
- 25 -: Stützgerüst
- 26 -: Draht
- 27 -: Längenabschnitt
- 28 -: Mantel
- 29 -: Draht
- 30 -: Längenabschnitt
- 31 -: Draht
- 32 -: Schenkel
- 33 -: Schenkel
- 34 -: Schenkel
- 35 -: Kante
- 36 -: Draht
- 37 -: Mantel
- 36 -: Umfang von 37
- 39 -: Vorsprung
- 40 -: Biegung
- 41 -: Schenkel
- 42 -: Schenkel
- 43 -: Draht
- 44 -: Schenkel
- 45 -: Schenkel
- 46 -: Sollbiegestelle
- 47 -: Sollbiegestelle
- 48 -: Spitze
- 49 -: Schenkelbereich
- 50 -: Schenkelbereich
- 51 -: Draht
- 52 -: Sollbiegestelle
- 53 -: Sollbiegestelle
- 54 -: Abschnitt
- 55 -: Abschnitt

- A -: Endpunkt
- B -: Knickpunkt
- C -: Knickpunkt
- D -: Endpunkt
- α -: Winkel
- β -: Winkel
- γ' -: Winkel
- γ'' -: Winkel
- δ' -: Winkel
- δ'' -: Winkel
- µ -: Winkel
- τ -: Winkel
- X -: Länge von 1
- L -: Längsachse von 24
- R -: Verzahnungsmaß
- PF1 -: Pfeil
- PF2 -: Pfeil
- PF3 -: Pfeil
- PF4 -: Pfeil
- PF5 -: Pfeil

## Patentansprüche

1. Platzhalter zum Anordnen in einer Körperröhre, der ein in einen hohlzylindrischen Mantel (24, 28, 37) aus mindestens bei Körpertemperatur begrenzt elastischem Kunststoff eingebettetes flexibles Stützgerüst (25) aus mindestens zwei jeweils zick-zack-förmig geformten Drähten, aufweist, wobei jeder Draht (1, 5, 12-18, 26, 29, 31, 36, 43, 51) mindestens drei Schenkel (2-4, 6-ε, 19-22, 32-34, 41, 42, 44, 45) aufweist, von denen der mittlere Schenkel (3, 7, 20, 21, 23) mit den beiden angrenzenden Schenkeln (2, 4, 6, 8, 32, 34) jeweils einen spitzen Winkel (α, β, γ', γ", δ', δ", µ, τ) einschließt,**dadurch gekennzeichnet, daß** die Drähte (25), sich parallel zur Längsachse (L) des Mantels (24, 28, 37) erstrecken und umfangsseitig des Mantels (24, 28, 37) zueinander versetzt sind.

2. Platzhalter nach Anspruch 1, **dadurch gekennzeichnet, daß** der Spitze Winkel (α, β, γ', γ", τ) kleiner als 45° ist.

3. Platzhalter nach Anspruch 1, **dadurch gekennzeichnet, daß** der spitze Winkel (δ' , δ", µ) größer oder gleich 45° und kleiner oder gleich 90° ist.

4. Platzhalter nach einem der Ansprüche 1 bis 3, **dadurch gekennzeichnet, daß** zwei umfangsseitig des Mantels (24, 28, 37) einander benachbarte Drähte (26, 29, 31) verzahnungsartig ineinandergreifen.

5. Platzhalter nach einem der Ansprüche 1 bis 4, **dadurch gekennzeichnet, daß** die benachbarten Schenkel (6, 7, 8) über gerundete Längenabschnitte (9, 10, 11, 27, 30) miteinander verbunden sind.

6. Platzhalter nach einem der Ansprüche 1 bis 5, **dadurch gekennzeichnet, daß** am Mantel (37) über den Umfang (38) verteilt Vorsprünge (39) vorgesehen sind.

7. Platzhalter nach einem der Ansprüche 1 bis 6, **dadurch gekennzeichnet, daß** jeder Vorsprung (39) durch zwei Schenkel (41, 42) miteinander verbindende, über den Umfang (38) vorstehende Längenabschnitte (40) gebildet ist.

8. Platzhalter nach einem der Ansprüche 1 bis 7, **dadurch gekennzeichnet, daß** die Drähte (1, 5, 12-18, 26, 29, 31, 36, 43, 51) aus Metall gebildet sind.

9. Platzhalter nach einem der Ansprüche 1 bis 8, **dadurch gekennzeichnet, daß** die Drähte (1, 5, 12-18, 26, 29, 31, 36, 43, 51) aus Stahl und der Mantel (24, 28, 37) aus einem Elastomer, insbesondere Silikon, bestehen.

10. Platzhalter nach einem der Ansprüche 1 bis 8, **dadurch gekennzeichnet, daß** die Drähte (1, 5, 12-18, 26, 29, 31, 36, 43, 51) aus einem Memory-Metall, insbesondere Nitinol, und der Mantel (24, 28, 37) aus einem Elastomer, insbesondere Latey, bestehen.

11. Platzhalter nach einem der Ansprüche 1 bis 8, **dadurch gekennzeichnet, daß** der Mantel (24, 28, 37) aus einem Memory-Elastomer besteht.

12. Platzhalter nach einem der Ansprüche 1 bis 8, **dadurch gekennzeichnet, daß** die Drähte (1, 5, 12-18, 26, 29, 31, 36, 43, 51) aus einem Memory-Metall, insbesondere Nitinol, und der Mantel (24, 28, 37) aus einem Memory-Elastomer, insbesondere auf Polyurethanbasis, bestehen.

13. Platzhalter nach einem der Ansprüche 1 bis 7, **dadurch gekennzeichnet, daß** die Drähte (1, 5, 12-18, 26, 29, 31, 36, 43, 51) aus Kunststoff bestehen.

14. Platzhalter nach einem der Ansprüche 1 bis 7, **dadurch gekennzeichnet, daß** die Drähte (1, 5, 12-18, 26, 29, 31, 36, 43, 51) aus Kunststoff und der Mantel (24, 28, 37) aus einem Memory-Elastomer bestehen.

15. Platzhalter nach einem der Ansprüche 1 bis 14, **dadurch gekennzeichnet, daß** die Drähte (43, 51) Sollbiegestellen (46, 47, 52, 53) aufweisen.

## Claims

1. A stent for fitting in a body tube, comprising a flexible supporting framework (25) consisting of at least two wires each of zigzag shape embedded in a hollow cylindrical jacket (24, 28, 37) of plastic having limited elasticity at least at body temperature, each wire (1, 5, 12-18, 26, 29, 31, 36, 43, 51) comprising at least three limbs (2-4, 6-8, 19-22, 32-34, 41, 42, 44, 45), of which the middle limb (3, 7, 20, 21, 23) together with the two adjoining limbs (2, 4, 6, 8, 32, 34) in each case includes an acute angle (α, β, γ', γ'', δ', δ'', µ, τ ), **characterised in that** the wires (25) extend parallel to the longitudinal axis (L) of the jacket (24, 28, 37) and are offset from one another peripherally of the jacket (24, 28, 37).

2. A stent according to claim 1, **characterised in that** the acute angle (α, β, γ', γ'', τ) is less than 45°.

3. A stent according to claim 1, **characterised in that** the acute angle (δ', δ'', µ) is larger than or equal to 45° and less than or equal to 90°.

4. A stent according to any one of claims 1 to 3, **characterised in that** two wires (26, 29, 31) adjacent one another peripherally of the jacket (24, 28, 37) interengage after the style of teeth.

5. A stent according to any one of claims 1 to 4, **characterised in that** the adjacent limbs (6, 7, 8) are interconnected via radiused length portions (9, 10, 11, 27, 30).

6. A stent according to any one of claims 1 to 5, **characterised in that** projections (39) are provided on the jacket (37) and are distributed over the periphery (38).

7. A stent according to any one of claims 1 to 6, **characterised in that** each projection (39) is formed by length portions (40) projecting beyond the periphery (38) and interconnecting two links (41, 42).

8. A stent according to any one of claims 1 to 7, **characterised in that** the wires (1, 5, 12-18, 26, 29, 31, 36, 43, 51) are formed of metal.

9. A stent according to any one of claims 1 to 8, **characterised in that** the wires (1, 5, 12-18, 26, 29, 31, 36, 43, 51) consist of steel and the jacket (24, 28, 37) consists of an elastomer, particularly silicone.

10. A stent according to any one of claims 1 to 8, **characterised in that** the wires (1, 5, 12-18, 26, 29, 31, 36, 43, 51) consist of a memory metal, more particularly nitinol, and the jacket (24, 28, 37) consists of an elastomer, particularly latex.

11. A stent according to any one of claims 1 to 8, **characterised in that** the jacket (24, 28, 37) consists of a memory elastomer.

12. A stent according to any one of claims 1 to 8, **characterised in that** the wires (1, 5, 12-18, 26, 29, 31, 36, 43, 51) consist of a memory metal, particularly nitinol, and the jacket (24, 28, 37) consists of a memory elastomer, particularly on a polyurethane basis.

13. A stent according to any one of claims 1 to 7, **characterised in that** the wires (1, 5, 12-18, 26, 29, 31, 36, 43, 51) consist of plastic.

14. A stent according to any one of claims 1 to 7, **characterised in that** the wires, (1, 5, 12-18, 26, 29, 31, 36, 43, 51) consist of (plastic and the jacket (24, 28, 37) consists of, a memory elastomer.

15. A stent according to any one of claims 1 to 14, **characterised in that** the wires (43, 51) have intentional bending points (46, 47, 52, 53).

## Revendications

1. Extenseur destiné à être mis en place à l'intérieur d'un vaisseau corporel, lequel comporte insérée dans une enveloppe cylindrique creuse (24,28,37), en matière synthétique élastique limitée) au moins à la température corporelle, une structure de support souple (25) constituée d'au moins deux fils formés respectivement en zigzag, , où chaque fil (1,5,12-18,26,29,31,36,43,51) présentant au moins trois branches (2-4,6-8,19-22,32-34,41,42,44,45) dont la branche médiane (3,7,20,21,23) inscrit respectivement avec les deux branches limitrophes (2,4,6,8,32,34) un angle aigu (α,β,γ',γ",δ',δ",µ,τ),
**caractérisé en ce que** les fils (25) s'étendent parallèlement à l'ax longitudinal (L) de l'enveloppe (24,28,37) et sont décalés entre eux sur le côté périphérique de l'enveloppe (24,28,37).

2. Extenseur selon la revendication 1, **caractérisé en ce que** l'angle aigu (α,β,γ',γ"τ) est inférieur à 45°.

3. Extenseur selon la revendication 1, **caractérisé en ce que** l'angle aigu (δ', δ",µ) est supérieur ou égal à 45° et inférieur ou égal à 90°.

4. Extenseur selon l'une des revendications 1 à 3,
**caractérisé en ce que** deux fils contigus (26,29,31) sur le côté périphérique de l'enveloppe (24,28,37) s'engrènent l'un dans l'autre à la manière d'une denture.

5. Extenseur selon l'une des revendications 1 à 4,
**caractérisé en ce que** les branches contigues (6,7,8) sont raccordées entre elles par le biais de sections longitudinales arrondies (9,10,11,27,30).

6. Extenseur selon l'une des revendications 1 à 5,
**caractérisé en ce que** sur l'enveloppe (37) sont prévues des saillies (39) réparties sur la périphérie (38).

7. Extenseur selon l'une des revendications 1 à 6,
**caractérisé en ce que** chaque saillie (39), , est formée, à travers deux branches (41,42) raccordées entre elles, par des sections longitudinales (40) se projetant sur la périphérie (38),

8. Extenseur selon l'une des revendications 1 à 7,
**caractérisé en ce que** les fils (1,5,12-18,26,29,31,36,43,51) sont réalisés en métal.

9. Extenseur selon l'une des revendications 1 à 8, **caractérisé en ce que** les fils (1,5,12-18,26,29,31,36,43,51) consistent en acier et l'enveloppe (24,28,37) consiste en un élastomère, en particulier de la silicone.

10. Extenseur selon l'une des revendications 1 à 8,
**caractérisé en ce que** les fils (1,5,12-18,26,29,31,36,43,51) consistent en un métal à mémoire, en particulier du nitinol, et l'enveloppe (24,28,37) consiste en un élastomère, en particulier du latex.

11. Extenseur selon l'une des revendications 1 à 8,
**caractérisé en ce que** l'enveloppe (24,28,37) consiste en un élastomère à mémoire.

12. Extenseur selon l'une des revendications 1 à 8,
**caractérisé en ce que** les fils (1,5,12-18,26,29,31,36,43,51) consistent en un métal à mémoire, en particulier du nitinol, et l'enveloppe (24,28,37) consiste en un élastomère à mémoire, en particulier à base de polyuréthane.

13. Extenseur selon l'une des revendications 1 à 7,
**caractérisé en ce que** les fils (1,5,12-18,26,29,31,36,43,51) consistent en une matière synthétique.

14. Extenseur selon l'une des revendications 1 à 7,
**caractérisé en ce que** les fils (1,5,12-18,26,29,31,36,43,51) consistent en matière synthétique et l'enveloppe (24,28,37) consiste en un élastomère à mémoire.

15. Extenseur selon l'une des revendications 1 à 14,
**caractérisé en ce que** les fils (43,51) présentent des zones de flexion nominale (46,47,52,53).
